# EUROPEAN PATENT APPLICATION

(11) **EP 4 722 218 A1**
(43) Date of publication of application: **08.04.2026**
(21) Application number: 24815563.2
(22) Date of filing: 30.05.2024
(51) Int. Cl.: C07D 487/14, A61K 31/519, A61P 35/00

(54) **CRYSTAL OF QUINOLINE-SUBSTITUTED COMPOUND**

(30) Priority: 31.05.2023 JP 2023090561
(71) Applicant: Taiho Pharmaceutical Co., Ltd., Chiyoda-ku, Tokyo 101-8444 (JP)
(72) Inventor: UNO, Takao, Tsukuba-shi, Ibaraki 300-2611 (JP); TAKEDA, Daisuke, Kodama-gun, Saitama 367-0241 (JP)
(74) Representative: Wächtershäuser & Hartz Patentanwaltspartnerschaft mbB
(86) International application number: PCT/JP2024/019849
(87) International publication number: WO 2024/248083

(57) **Abstract**

An object is to provide a crystal of a compound having an EGFR inhibition ability or a salt thereof, the crystal being satisfactory in one or more of the following characteristics: stability (in terms of heat or purity) and/or low hygroscopicity. According to one aspect of the present invention, there is provided a crystal form of (S)-N-(4-amino-6-methyl-5-(quinolin-3-yl)-8,9-dihydropyrimido[5,4-b]lindolizin-8-yl)acrylamide having a peak at a predetermined diffraction angle (2θ±0.2°) in its powder X-ray diffraction spectrum.

## Description

### Technical Field

The present invention relates to, for example, a crystal of a quinoline-substituted compound and a crystallization method of the compound.

### Background Art

An epidermal growth factor receptor (EGFR) is a receptor-type tyrosine kinase. The EGFR binds to an epidermal growth factor (EGF) serving as a ligand to exhibit a physiological function in a normal tissue, and contributes to, for example, proliferation and apoptosis inhibition in an epidermal tissue (Non-patent Document 1).

The EGFR also serves as an oncogene, and the amplification of an EGFR gene, and the high expression and mutation of an EGFR protein have been known in various kinds of cancers, such as head and neck cancer, breast cancer, colon cancer, esophageal cancer, pancreatic cancer, lung cancer, ovarian cancer, renal cancer, bladder cancer, skin cancer, and a brain tumor (Non-patent Document 2). In Japan and the Western countries, about 170 to about 360 per 100,000 persons die of cancer annually, and hence the cancer ranks high in the causes of death (Non-patent Document 3). The yearly number of deaths due to lung cancer out of cancers reaches as high as about 1,400,000 in the whole world, and non-small cell lung cancer accounts for 80% or more of the lung cancers. Accordingly, the development of an effective treatment method for non-small cell lung cancer has been desired (Non-patent Document 4).

As an antitumor agent having EGFR inhibition activity, in Patent Document 1, there is a description of a compound represented by the following formula (I).

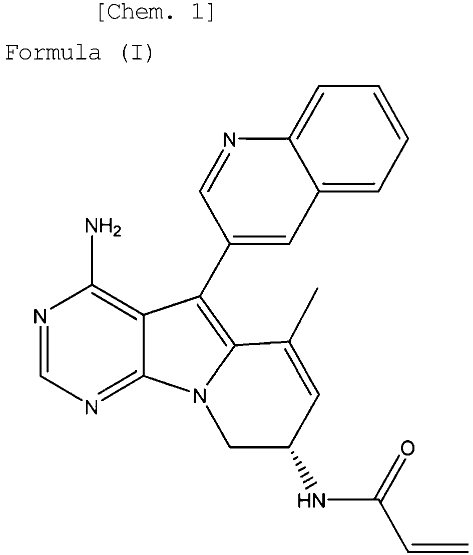

The quinoline-substituted compound is (S)-N-(4-amino-6-methyl-5-(quinolin-3-yl)-8,9-dihydropyrimido[5,4-b]indolizin-8-yl)acrylamide (in this description, (S)-N-(4-amino-6-methyl-5-(quinolin-3-yl)-8,9-dihydropyrimido[5,4-b]indolizin-8-yl)acrylamide is hereinafter also referred to as "compound (1)").

In general, when a compound is used as an active ingredient of a drug, the chemical and physical stability of the compound is required for stably holding its quality. Accordingly, the resultant compound desirably has a stable crystal form. However, a crystal may have crystal polymorphs that are different from each other in intracrystalline molecular arrangement even in one and the same molecule. It has been known that when crystal forms are different from each other, peaks to be obtained by powder X-ray diffractometry (XRD) measurement are different from each other. Further, it has been known that the respective crystal forms are different from each other in physical properties, such as solubility, stability, and hygroscopicity. In drug development, it has been required to find out an optimum crystal form from the viewpoints of, for example, quality and production.

In Patent Document 1, there is a description that the compound (1) is obtained by: adding diisopropylethylamine and a solution of acryloyl chloride in acetonitrile to a solution of a mixture of (S)-6-methyl-5-(quinolin-3-yl)-8,9-dihydropyrimido[5,4-b]indolizine-4,8-diamine and (S)-6-methylene-5-(quinolin-3-yl)-6,7,8,9-tetrahydropyrimido[5,4-b]indolizine-4,8-diamine in acetonitrile and water; stirring the mixture; extracting with water, a saturated sodium bicarbonate solution, and ethyl acetate after the completion of reaction; and then drying and purifying the extract. However, there is no description about what crystal form the compound (1) obtained by the method has. Further, there are no specific disclosures about what crystal form the compound (1) or a salt thereof may form, and what physical properties the crystal form has.

### Citation List

### Patent Document

Patent Document 1: WO 2015/025936 A1

### Non-patent Document

Non-patent Document 1: Nature Rev. Cancer, vol. 6, pp 803-811 (2006)
Non-patent Document 2: J. Clin. Oncol., vol. 19, 32s-40s (2001)
Non-patent Document 3: Ministry of Internal Affairs and Communications Statistics Bureau of Japan Home Page/Statistical Data/World Statistics "World Statistics 2022," Chapter 14. Daily Lives of People and Social Security, 14-1. Cause-specific Mortality Rate
Non-patent Document 4: Lung Cancer, vol. 69, pp 1-12 (2010)

### Summary of Invention

### Technical Problem

When a compound is used as an active pharmaceutical ingredient of a drug, a stable crystal form of the compound is desirably formed for stably holding its quality and/or for facilitating its storage management. Further, the crystal form of the compound preferably has low hygroscopicity and the like. However, it is difficult to predict whether or not a specific compound or a salt thereof forms a crystal, and what crystal form is excellent in physical properties such as stability.

In view of such circumstances as described above, there has been required a crystal of a compound having an EGFR inhibition ability or a salt thereof, the crystal being satisfactory in one or more of the characteristics such as thermal stability, storage stability, and/or low hygroscopicity. An object of the present disclosure is to provide a compound (1) having one or more of the characteristics such as thermal stability, storage stability, and/or low hygroscopicity. Another object of the present disclosure is to provide a crystal of the compound (1) useful as a drug or a drug substance. Another object of the present disclosure is to provide a method for producing a crystal of the compound (1).

### Solution to Problem

The inventors of the present invention have made extensive investigations, and as a result, have obtained a free-base crystal form of the compound (1) having satisfactory properties in terms of the following characteristics: thermal stability, storage stability, and/or low hygroscopicity.

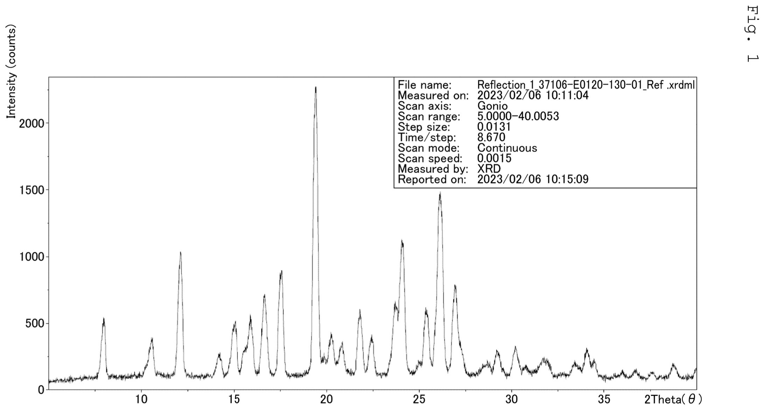

That is, the present invention provides, for example, the following items [1] to [16].
[1] A type I crystal of (S)-N-(4-amino-6-methyl-5-(quinolin-3-yl)-8,9-dihydropyrimido[5,4-b]indolizin-8-yl)acrylamide, the crystal having, in a powder X-ray diffraction spectrum thereof measured with a CuKα characteristic X-ray, peaks at 3 or more diffraction angles (2θ±0.2°) selected from 8.0°, 10.6°, 12.2°, 15.1°, 16.6°, 17.6°, 19.4°, 21.7°, and 26.1°.
[2] A type I crystal of (S)-N-(4-amino-6-methyl-5-(quinolin-3-yl)-8,9-dihydropyrimido[5,4-b]indolizin-8-yl)acrylamide, the crystal having, in a powder X-ray diffraction spectrum thereof measured with a CuKα characteristic X-ray, peaks at 5 or more diffraction angles (2θ±0.2°) selected from 8.0°, 10.6°, 12.2°, 15.1°, 16.6°, 17.6°, 19.4°, 21.7°, and 26.1°.
[3] A type I crystal of (S)-N-(4-amino-6-methyl-5-(quinolin-3-yl)-8,9-dihydropyrimido[5,4-b]indolizin-8-yl)acrylamide, the crystal having, in a powder X-ray diffraction spectrum thereof measured with a CuKα characteristic X-ray, peaks at 7 or more diffraction angles (2θ±0.2°) selected from 8.0°, 10.6°, 12.2°, 15.1°, 16.6°, 17.6°, 19.4°, 21.7°, and 26.1°.
[4] A type I crystal of (S)-N-(4-amino-6-methyl-5-(quinolin-3-yl)-8,9-dihydropyrimido[5,4-b]indolizin-8-yl)acrylamide, the crystal having a powder X-ray diffraction spectrum substantially the same as a powder X-ray diffraction spectrum measured with a CuKα characteristic X-ray, the spectrum being shown in FIG. 1.
[5] The crystal according to any one of Items [1] to [4], wherein the crystal has an endothermic peak (peak top value) determined by thermogravimetry-differential thermal analysis at around 244°C.
[6] The crystal according to any one of Items [1] to [5], wherein the crystal has a crystal purity of 50 wt% or more (preferably 75 wt% or more, more preferably 80 wt% or more, still more preferably 95 wt% or more).
[7] The crystal according to any one of Items [1] to [6], wherein the crystal has a chemical purity of 90% or more (preferably 95% or more, more preferably 97% or more, still more preferably 98% or more).
[8] A pharmaceutical composition including the crystal of any one of Items [1] to [7].
[9] A pharmaceutical composition including: the crystal of any one of Items [1] to [7]; and a pharmaceutically acceptable carrier.
[10] An antitumor agent including the crystal of any one of Items [1] to [7].
[11] A method of producing the type I crystal of (S)-N-(4-amino-6-methyl-5-(quinolin-3-yl)-8,9-dihydropyrimido[5,4-b]indolizin-8-yl)acrylamide of any one of Items [1] to [7], the method including a step of stirring (S)-N-(4-amino-6-methyl-5-(quinolin-3-yl)-8,9-dihydropyrimido[5,4-b]indolizin-8-yl)acrylamide in a solvent containing at least one kind selected from the group consisting of: a lower alcohol; an aprotic polar solvent; and water (in one kind of solvent or in a mixed solvent of two or more kinds of solvents).
[12] A method of producing the type I crystal of (S)-N-(4-amino-6-methyl-5-(quinolin-3-yl)-8,9-dihydropyrimido[5,4-b]indolizin-8-yl)acrylamide of any one of Items [1] to [7], the method including a step of dissolving and crystallizing (S)-N-(4-amino-6-methyl-5-(quinolin-3-yl)-8,9-dihydropyrimido[5,4-b]indolizin-8-yl)acrylamide in a mixed solvent of a lower alcohol and water, or a mixed solvent of an aprotic polar solvent and water.
[13] The method of producing the type I crystal of (S)-N-(4-amino-6-methyl-5-(quinolin-3-yl)-8,9-dihydropyrimido[5,4-b]indolizin-8-yl)acrylamide according to Item [12], the method including a step of dissolving and crystallizing (S)-N-(4-amino-6-methyl-5-(quinolin-3-yl)-8,9-dihydropyrimido[5,4-b]indolizin-8-yl)acrylamide in a mixed solvent of ethanol and water (the ratio "ethanol:water" is from 4:1 to 0.25:1, preferably from 3:1 to 0.25:1, more preferably from 2:1 to 0.25:1, still more preferably from 1.5:1 to 0.5:1, still further more preferably from 1:1 to 0.5:1, particularly preferably from 0.67:1 to 0.5:1, most preferably 0.5:1), or a mixed solvent of acetonitrile and water (the ratio "acetonitrile:water" is from 3:1 to 0.15:1, preferably from 1.5:1 to 0.15:1, more preferably from 1:1 to 0.15:1, still more preferably from 0.67:1 to 0.33:1, still further more preferably from 0.5:1 to 0.33:1, particularly preferably 0.5:1).
[14] The method of producing the type I crystal of (S)-N-(4-amino-6-methyl-5-(quinolin-3-yl)-8,9-dihydropyrimido[5,4-b]indolizin-8-yl)acrylamide according to Item [12] or [13], wherein (S)-N-(4-amino-6-methyl-5-(quinolin-3-yl)-8,9-dihydropyrimido[5,4-b]indolizin-8-yl)acrylamide has a chemical purity of 90% or more (preferably 95% or more, more preferably 97% or more, still more preferably 98% or more, still further more preferably 99% or more).
[15] A method of treating a tumor, the method including orally administering an effective dose of the crystal of any one of Items [1] to [7] to a subject in need thereof.
[16] A use of the crystal of any one of Items [1] to [7] for production of an antitumor agent for oral administration.

### Advantageous Effects of Invention

According to one aspect of the present invention, the crystal of the compound having an EGFR inhibition ability or the salt thereof, the crystal being satisfactory in terms of the characteristics such as thermal stability, storage stability, and/or low hygroscopicity, is provided.

### Brief Description of Drawings

[FIG. 1] FIG. 1 shows the powder X-ray diffraction spectrum of the type I crystal of the compound (1) obtained in Example 1 (an axis of ordinate indicates an intensity (counts) and an axis of abscissa indicates a diffraction angle (2θ)).
[FIG. 2] FIG. 2 shows the results of the thermogravimetry-differential thermal analysis (TG-DTA) of the type I crystal of the compound (1) obtained in Example 1 (a left axis of ordinate indicates a weight (%) in a TG curve, a right axis of ordinate indicates a heat flux (µV) in a DTA curve, and an axis of abscissa indicates a temperature (°C)).
[FIG. 3] FIG. 3 shows the powder X-ray diffraction spectrum of the type I crystal of the compound (1) obtained in Example 2 (an axis of ordinate indicates an intensity (counts) and an axis of abscissa indicates a diffraction angle (2θ)).
[FIG. 4] FIG. 4 shows the powder X-ray diffraction spectrum of the solvate crystal type "b" of the compound (1) obtained in Comparative Example 1 (an axis of ordinate indicates an intensity (counts) and an axis of abscissa indicates a diffraction angle (2θ)).
[FIG. 5] FIG. 5 shows the results of the thermogravimetry-differential thermal analysis (TG-DTA) of the solvate crystal type "b" of the compound (1) obtained in Comparative Example 1 (a left axis of ordinate indicates a weight (%) in a TG curve, a right axis of ordinate indicates a heat flux (µV) in a DTA curve, and an axis of abscissa indicates a temperature (°C)).
[FIG. 6] FIG. 6 shows the powder X-ray diffraction spectrum of the amorphous solid of the compound (1) obtained in Reference Example 1 (an axis of ordinate indicates an intensity (counts) and an axis of abscissa indicates a diffraction angle (2θ)).
[FIG. 7] FIG. 7 shows the results of the thermogravimetry-differential thermal analysis (TG-DTA) of the amorphous solid of the compound (1) obtained in Reference Example 1 (a left axis of ordinate indicates a weight (%) in a TG curve, a right axis of ordinate indicates a heat flux (µV) in a DTA curve, and an axis of abscissa indicates a temperature (°C)).
[FIG. 8] FIG. 8 shows the results of the dynamic vapor sorption (DVS) test of the type I crystal of the compound (1) of Test Example 2.
[FIG. 9] FIG. 9 shows the results of the dynamic vapor sorption (DVS) test of the solvate crystal type "b" of the compound (1) of Test Example 2.
[FIG. 10] FIG. 10 shows the results of the dynamic vapor sorption (DVS) test of the amorphous solid of the compound (1) of Test Example 2.

### Description of Embodiments

The present invention relates to a crystal of (S)-N-(4-amino-6-methyl-5-(quinolin-3-yl)-8,9-dihydropyrimido[5,4-b]indolizin-8-yl)acrylamide represented by the following formula (I).

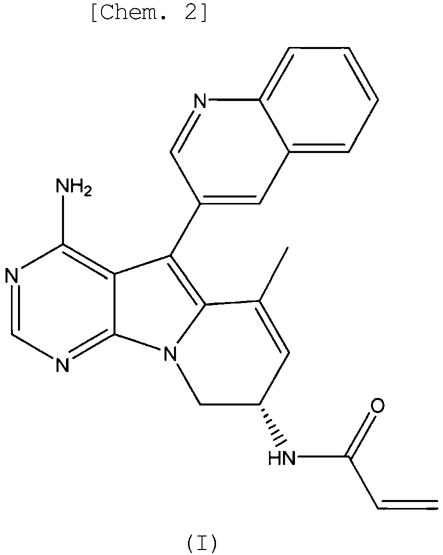

Specifically, the present invention relates to a crystal of the compound (1) (i.e., a type I crystal of the compound (1)). For example, the descriptions "type I" and "solvate crystal type "b"" as used herein are convenient names for distinguishing crystal forms, and the crystal according to the present invention is not limited by the names.

A crystal refers to a solid in which atoms or molecules are arranged to form a regular repeating structure, and is different from an amorphous solid free of any repeating structure. A crystal or an amorphous solid may be examined by a method, such as powder X-ray diffractometry (XRD) measurement, differential scanning calorimetry (DSC) measurement, thermogravimetry-differential thermal analysis (TG-DTA), or single crystal analysis. Crystal polymorphs refer to forms different from each other in intracrystalline atomic or molecular arrangement in one and the same molecule, and it has been known that peaks obtained by the XRD measurement differ from each other between the crystal polymorphs. In addition, it has been known that the respective crystal polymorphs are different from each other in, for example, solubility, oral absorptivity, and/or stability.

The terms "crystal" and "amorphous" as used herein are used in typical meanings, and whether or not a solid is a crystal can be recognized with an X-ray diffraction spectrum.

In a powder X-ray diffraction pattern, at the time of the recognition of crystal identity, a diffraction angle and an entire pattern are important in terms of data characteristics. The relative intensity of the powder X-ray diffraction pattern should not be strictly construed because the intensity may fluctuate to some extent owing to the direction of crystal growth, a particle size, measurement conditions, a state in which a measuring device is maintained, and a method of preparing a measurement sample. The phrase "powder X-ray diffraction spectrum substantially the same as a powder X-ray diffraction spectrum shown in a figure" as used herein means a powder X-ray diffraction spectrum that may be recognized as being the same as the powder X-ray diffraction spectrum shown in the figure by a person skilled in the art in consideration of a slight fluctuation in peak position or intensity. For example, the numerical value of the diffraction angle (2θ) may have a measurement error in the range of about ±0.2°.

The simple description "compound (1)" as used herein means (S)-N-(4-amino-6-methyl-5-(quinolin-3-yl)-8,9-dihydropyrimido[5,4-b]indolizin-8-yl)acrylamide, and is used in a meaning including both of "crystal" and "amorphous".

The description "crystal of the compound (1)" as used herein means a free-base crystal of the compound (1).

In this specification, such a crystal that a molecule (any other molecule for forming a salt or a cocrystal) except the compound (1) for forming the crystal is not identified means a free-base crystal of (S)-N-(4-amino-6-methyl-5-(quinolin-3-yl)-8,9-dihydropyrimido[5,4-b]indolizin-8-yl)acrylamide. The crystal is not limited to a single crystal form such as a type I, and may encompass a plurality of free-base crystals.

In this specification, a crystal of (S)-N-(4-amino-6-methyl-5-(quinolin-3-yl)-8,9-dihydropyrimido[5,4-b]indolizin-8-yl)acrylamide (compound (1)) with an acid means a salt crystal or a cocrystal with the acid. The salt crystal is a crystal obtained by the bonding of the compound (1) and a molecule of the acid through an ionic bond, and the cocrystal is a crystal obtained by the bonding of the compound (1) and a molecule of the acid through a nonionic interaction. In the present invention, the crystal of the compound (1) with the acid may be a salt crystal or a cocrystal, and encompasses both the meanings. For example, a crystal of (S)-N-(4-amino-6-methyl-5-(quinolin-3-yl)-8,9-dihydropyrimido[5,4-b]indolizin-8-yl)acrylamide with succinic acid means a crystal of a succinic acid salt of (S)-N-(4-amino-6-methyl-5-(quinolin-3-yl)-8,9-dihydropyrimido[5,4-b]indolizin-8-yl)acrylamide or a cocrystal of (S)-N-(4-amino-6-methyl-5-(quinolin-3-yl)-8,9-dihydropyrimido[5,4-b]indolizin-8-yl)acrylamide with succinic acid.

In general, examples of the salt of the pharmaceutically acceptable acid include many salts including salts of: inorganic acids, such as hydrochloric acid, hydrobromic acid, hydroiodic acid, sulfuric acid, nitric acid, and phosphoric acid; and organic acids, such as formic acid, acetic acid, propionic acid, oxalic acid, malonic acid, succinic acid, ascorbic acid, isoascorbic acid, mandelic acid, fumaric acid, aspartic acid, maleic acid, lactic acid, malic acid, hippuric acid, glutaric acid, adipic acid, citric acid, tartaric acid, carbonic acid, picric acid, methanesulfonic acid (mesylic acid), p-toluenesulfonic acid (p-tosic acid), and glutamic acid.

In this specification, the crystal may be any one of a hydrate or an anhydride.

A label body of the compound (1) or a salt thereof, that is, a compound obtained by substituting one or more atoms of the compound (1) or the salt of the compound (1) with a radioactive isotope or a nonradioactive isotope is also included in the present invention.

The crystal only needs to include the crystal of the compound (1), and may be a single crystal or a polymorphic mixture including the crystal of the compound (1) except the foregoing. Specifically, the purity of the crystal may be 40 wt% or more (i.e., 40 wt% or more thereof is a single crystal). The purity of the crystal is preferably 50 wt% or more (i.e., 50 wt% or more thereof is a single crystal), the purity of the crystal is more preferably 75 wt% or more (i.e., 75 wt% or more thereof is a single crystal), the purity of the crystal is more preferably 90 wt% or more (i.e., 90 wt% or more thereof is a single crystal), the purity of the crystal is still more preferably 95 wt% or more (i.e., 95 wt% or more thereof is a single crystal), the purity of the crystal is still more preferably 98 wt% or more (i.e., 98 wt% or more thereof is a single crystal), and the purity of the crystal is particularly preferably 99 wt% or more (i.e., 99 wt% or more thereof is a single crystal). The purity may be measured by performing analysis such as differential scanning calorimetry (DSC) measurement.

In this specification, a chemical purity is a purity at the time of measurement by high performance liquid chromatography (HPLC), and the description "chemical purity of the compound (1)" refers to the purity of the compound (1) at the time of measurement by the HPLC. At that time, the wavelength of a detector to be used in purity measurement may be appropriately set. Specifically, the chemical purity of the crystal of the compound (1) is preferably 90% or more, more preferably 95% or more, still more preferably 97% or more, particularly preferably 98% or more.

In this specification, an optical purity is a purity at the time of measurement with a polarimeter, and the description "optical purity of the compound (1)" refers to the purity of the compound (1) at the time of measurement with the polarimeter. At that time, the light source of an apparatus to be used in purity measurement may be appropriately selected.

Some error may occur in a numerical value obtained from a powder X-ray diffraction pattern depending on, for example, the direction of crystal growth, a particle size, and measurement conditions. Accordingly, in this specification, the numerical value of a diffraction angle (2θ) in the powder X-ray diffraction pattern may have a measurement error in the range of about ±0.2°. That is, the notation "diffraction angle (2θ±0.2°)" as used herein means that a measurement error of ±0.2° is allowable in the numerical value of the diffraction angle (2θ). For example, the description "8.0°" for the diffraction angle (2θ±0.2°) means that a diffraction angle of 8.0°±0.2° is allowable, and the description encompasses a diffraction angle of "from 7.8° to 8.2°." In addition, the value may be determined from Bragg's formula (2dsinθ=nλ), and changes with the wavelength at which the value is measured. That is, the substitution of a measurement wavelength into the above-mentioned formula can convert a diffraction angle into a value at another measurement wavelength. For example, when the numerical values of the diffraction angles (2θ) at a wavelength λ of a CuKα characteristic X-ray of 1.54 Å are 8.0° and 10.6°, respectively, and the values of "d" and "n" remain unchanged, the diffraction angles (2θ) at 0.75 Å are 3.9° and 5.2°, respectively.

The compound (1) to be used in a crystallization method of the present invention is, for example, a compound produced by a method described in Patent Document 1. At the time of the crystallization, a product that remains free from being recovered as a crystal after the synthesis of the compound (1), or a product recovered as a crystal (crude crystal) once thereafter may be used.

The term "room temperature" as used herein typically refers to from about 18°C to about 25°C.

The term "lower alcohol" as used herein refers to an alcohol having 1 to 5 carbon atoms that may be linear or have a branched chain. Examples thereof include methanol, ethanol, propanol, butanol, and pentanol.

The term "ketone-based solvent" as used herein means a solvent having a ketone structure in a molecule thereof. Examples thereof include acetone and methyl ethyl ketone.

The term "ester-based solvent" as used herein means a solvent having an ester structure in a molecule thereof. Examples thereof include ethyl acetate, isopropyl acetate, and butyl acetate.

The term "saturated hydrocarbon-based solvent" as used herein means a solvent formed of a saturated hydrocarbon that may be linear or have a branched chain. Examples thereof include hexane and heptane.

The term "ether-based solvent" as used herein means a solvent having an ether structure in a molecule thereof. Examples thereof include diethyl ether, ethyl methyl ether, diphenyl ether, ethylene oxide, tetrahydrofuran, 1,4-dioxane, and benzofuran.

An aprotic polar organic solvent means a solvent in which a proton that is ionized is absent. Examples thereof include acetonitrile, N,N-dimethylformamide (DMF), N,N-dimethylacetamide (DMA), N-methylpyrrolidone (NMP), chloroform, dichloromethane, and dimethyl sulfoxide (DMSO).

The temperature at which an endothermic peak in a simultaneous thermogravimetry-differential thermal analysis (TG-DTA) curve is measured may change with, for example, a temperature increase width per minute and the chemical purity of a sample, and the change typically means ±5.0°C. Accordingly, when the crystal according to the present invention is subjected to TG-DTA measurement, a width of ±5.0°C is taken into consideration as the error of its endothermic peak (peak top value). The term "around" to be used at that time means ±5.0°C.

One embodiment of the present invention relates to a type I crystal of the compound (1). The inventors of the present invention have found that the type I crystal of the compound (1) has the following properties advantageous in drug production: thermal stability; storage stability; low hygroscopicity; and the fact that the crystal can be obtained with high reproducibility.

In this specification, the thermal stability and storage stability of the crystal may each be evaluated by, for example, a solid stability test described in Test Example 1 to be described later. A change in chemical purity of the crystal of the compound (1) in one embodiment of the present invention after its storage under a condition described in Test Example 1 for 2 weeks or 4 weeks is preferably ±1.0% or less, more preferably ±0.5% or less, still more preferably ±0.1% or less. In addition, the crystal shows no change in crystal form when subjected to XRD measurement, and also shows no change in appearance (color tone) after its storage.

In this specification, the hygroscopicity of the crystal may be evaluated by a dynamic vapor sorption (DVS) test described in Test Example 2 to be described later. The weight increase ratio of the crystal of the compound (1) in one embodiment of the present invention when evaluated under conditions described in Test Example 2 is preferably less than 5%, more preferably less than 3%, still more preferably less than 2%.

One embodiment of the present invention provides an antitumor agent including the crystal of the compound (1) described above (i.e., the type I crystal of the compound (1)). In addition, one embodiment of the present invention provides a method of treating a tumor, the method including administering an effective dose of the crystal of the compound (1) described above (i.e., the type I crystal of the compound (1)) to a subject in need thereof. According to one embodiment of the present invention, there is provided a use of the crystal of the compound (1) (i.e., the type I crystal of the compound (1)) for the production of an antitumor agent. Further, according to one embodiment of the present invention, there is provided the crystal of the compound (1) (i.e., the type I crystal of the compound (1)) for use in the treatment of a tumor. According to one embodiment of the present invention, there is provided a method for producing a crystal of the compound (1) described above (i.e., the type I crystal of the compound (1)).

The type I crystal of the compound (1) in one embodiment of the present invention has such properties advantageous in drug production as described below as compared to any other crystal of the compound (1) such as a solvate crystal type "b": thermal stability; storage stability; low hygroscopicity; and excellent reproducibility with which the crystal is obtained.

### (S)-N-(4-Amino-6-methyl-5-(quinolin-3-yl)-8,9-dihydropyrimido[5,4-b]indolizin-8-yl)acrylamide (Compound (1))

The compound (1) is a compound having the following structure, the compound being disclosed as a compound having an excellent EGFR-inhibiting action in Patent Document 1.

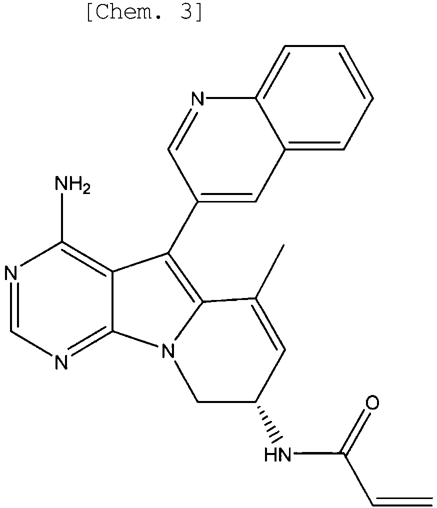

Although the compound (1) to be used in the present invention is not particularly limited, for example, compounds produced by a production method described in Patent Document 1 and a known production method may each be used. A crystal form may be produced by using the compound (1).

### Method of producing Type I Crystal of (S)-N-(4-Amino-6-methyl-5-(quinolin-3-yl)-8,9-dihydropyrimido[5,4-b]indolizin-8-yl)acrylamide (Compound (1))

The type I crystal of the compound (1) may be produced by each of a slurry method, a dissolution-crystallization method, and any other known crystal production method. A method of producing the type I crystal of the compound (1) including using each of the slurry method and the dissolution-crystallization method is described in detail below, though the present invention is not limited to the production method.

### Slurry Method

In the present invention, the slurry method is a method including: adding a solvent to a container storing the compound (1) produced by the production method described in Patent Document 1 or a known production method; stirring the mixture under a suspended state for from about several hours to about several days; filtering the mixture; and then drying the resultant crystal to produce a crystal.

The crystal may be obtained by appropriately adjusting parameters, such as the kind of the solvent, the temperature of the solvent at the time of the stirring, and a stirring time.

Any solvent may be used as the solvent as long as (S)-N-(4-amino-6-methyl-5-(quinolin-3-yl)-8,9-dihydropyrimido[5,4-b]indolizin-8-yl)acrylamide can maintain the suspended state in the above-mentioned stirring step. Examples thereof include a lower alcohol (e.g., methanol, ethanol, 1-propanol, or 2-propanol), a ketone-based solvent (e.g., methyl ethyl ketone or acetone), an ester-based solvent (e.g., ethyl acetate, isopropyl acetate, or butyl acetate), a saturated hydrocarbon-based solvent (e.g., hexane), an ether-based solvent (e.g., tetrahydrofuran, 1,4-dioxane, methyl-tert-butyl ether, or diisopropyl ether), an aprotic polar solvent (e.g., acetonitrile or chloroform), an aryl halide (e.g., chlorobenzene), and water. Of those, for example, a lower alcohol, an aprotic polar solvent, or water is preferred. Those solvents may be used alone or in combination thereof at an arbitrary ratio. A mixed solvent of two or more kinds thereof is, for example, a solvent obtained by mixing methanol and water at 1:1, ethanol and water at 1:1, or ethanol and diisopropyl ether at 1:1. A solvent formed only of acetonitrile, or a mixed solvent obtained by mixing ethanol and water at 1:1 is preferred. The solvent may be used in an amount of from 0.001 mL to 0.03 mL with respect to 1 mg of the compound (1). The amount is preferably from 0.002 mL to 0.02 mL, more preferably from 0.003 mL to 0.01 mL, still more preferably 0.005 mL.

Although the temperature of the solvent may be appropriately adjusted, the stirring may be performed at, for example, from 20°C to 60°C. The temperature is preferably from 25°C to 50°C, more preferably from 30°C to 50°C, still more preferably from 40°C to 50°C, most preferably 50°C. To precipitate the crystal of the compound (1), the stirred mixture is preferably left standing to cool after the stirring at the above-mentioned temperature. The mixture may be left standing to cool to a temperature of, for example, from 10°C to 40°C. The temperature is preferably from 15°C to 30°C, more preferably room temperature.

Although the stirring time is appropriately adjusted, the stirring may be performed for, for example, from 1 hour to 72 hours. The time is preferably from 4 hours to 60 hours, more preferably from 8 hours to 48 hours, still more preferably from 12 hours to 36 hours, most preferably 24 hours.

The precipitated crystal may be isolated and purified from, for example, the suspension of the crystal by known separation and purification means, such as filtration, washing with water or an organic solvent, or drying under reduced pressure. Examples of the organic solvent to be used in the washing include a lower alcohol, acetone, and acetonitrile.

### Dissolution-crystallization Method

In the present invention, the dissolution-crystallization method is a method including: dissolving the compound (1) produced by the production method described in Patent Document 1 or a known production method in a solvent; stirring the solution or slurry under a warming or cooling condition for from about several hours to about several days; filtering the solid; and then drying the resultant crystal to produce a crystal. In addition, in the method, a seed crystal may be added as required.

In this method, the crystal may be obtained by appropriately adjusting parameters, such as the kind of the solvent, the temperature of the solvent at the time of the stirring, and a stirring time.

The solvent is not particularly limited as long as (S)-N-(4-amino-6-methyl-5-(quinolin-3-yl)-8,9-dihydropyrimido[5,4-b]indolizin-8-yl)acrylamide is dissolved therein, and can be present under a stable state without the advance of its decomposition or the like in the solution. The solvent is, for example, a mixed solvent of a lower alcohol (e.g., methanol, ethanol, 1-propanol, or 2-propanol) and water, or a mixed solvent of an aprotic polar solvent (e.g., acetonitrile, DMF, DMA, NMP, or DMSO) and water. Those solvents may be used in combination thereof at an arbitrary ratio as long as the compound (1) is dissolved therein. For example, methanol and water may be used at a ratio of from 4:1 to 0.5:1. The ratio is preferably from 3:1 to 1:1, more preferably from 2:1 to 1:1, still more preferably from 1.5:1 to 1:1, particularly preferably 1:1. When ethanol and water are used as a mixture, ethanol and water may be used at a ratio of from 4:1 to 0.25:1. The ratio is preferably from 3:1 to 0.25:1, more preferably from 2:1 to 0.25:1, still more preferably from 1.5:1 to 0.5:1, still further more preferably from 1:1 to 0.5:1, particularly preferably from 0.67:1 to 0.5:1, most preferably 0.5:1. When 2-propanol and water are used as a mixture, 2-propanol and water may be used at a ratio of from 4:1 to 0.25:1. The ratio is preferably from 2:1 to 0.25:1, more preferably from 1.5:1 to 0.25:1, still more preferably from 1:1 to 0.5:1, still further more preferably from 0.67:1 to 0.5:1, particularly preferably 0.5:1. When 1-propanol and water are used as a mixture, 1-propanol and water may be used at a ratio of from 2:1 to 0.15:1. The ratio is preferably from 1.5:1 to 0.15:1, more preferably from 1:1 to 0.25:1, still more preferably from 0.67:1 to 0.33:1, still further more preferably from 0.5:1 to 0.33:1, particularly preferably 0.33:1. When acetonitrile and water are used as a mixture, acetonitrile and water may be used at a ratio of from 3:1 to 0.15:1. The ratio is preferably from 1.5:1 to 0.15:1, more preferably from 1:1 to 0.15:1, still more preferably from 0.67:1 to 0.33:1, still further more preferably from 0.5:1 to 0.33:1, particularly preferably 0.5:1. A preferred solvent is a mixed solvent of ethanol and water, or a mixed solvent of acetonitrile and water, and a more preferred solvent is a mixed solvent of ethanol and water. The ratio of the mixed solvent is as described above.

The solvent may be used in an amount of from 0.005 mL to 0.080 mL with respect to 1 mg of the compound (1). The amount is preferably from 0.015 mL to 0.060 mL, more preferably from 0.025 mL to 0.045 mL, still more preferably 0.035 mL.

Although the temperature of the solvent at the time of the dissolution of the compound (1) may be appropriately adjusted, the dissolution may be performed at, for example, from 20°C to 80°C. The temperature is preferably from 20°C to 60°C, more preferably from 30°C to 60°C, still more preferably from 45°C to 60°C, most preferably 55°C. In addition, to precipitate the crystal of the compound (1), the stirred mixture is preferably left standing to cool after the stirring at the above-mentioned temperature. The mixture may be left standing to cool to a temperature of, for example, from 10°C to 40°C. The temperature is preferably from 15°C to 30°C, more preferably room temperature.

Although the stirring time may be appropriately adjusted, the stirring may be performed for, for example, from 1 hour to 72 hours. The time is preferably from 5 hours to 60 hours, more preferably from 12 hours to 48 hours, still more preferably from 16 hours to 24 hours, most preferably 20 hours.

The precipitated crystal may be isolated and purified from, for example, the suspension of the crystal by known separation and purification means, such as filtration, washing with water or an organic solvent, or drying under reduced pressure. Examples of the organic solvent to be used in the washing include a lower alcohol, acetone, and acetonitrile.

When the crystal is produced by the dissolution-crystallization method, the production may be performed by adding the target crystal as a seed crystal to the above-mentioned solvent.

In each of the crystal production methods, the production of the crystal requires the maintenance of a state in which the compound (1) is saturated or oversaturated with respect to the solvent for a certain time period (e.g., from 1 hour to 72 hours, preferably from 4 hours to 60 hours, more preferably from 8 hours to 48 hours, still more preferably from 12 hours to 36 hours). A short time period (e.g., 30 minutes or less, less preferably from 1 second to 20 minutes, still less preferably from 30 seconds to 15 minutes) for which the saturated or oversaturated state is maintained is not preferred because the crystal cannot be produced, and hence an amorphous state is liable to be established.

The crystal purity of the type I crystal of the compound (1) produced by the dissolution-crystallization method is 90 wt% or more. The purity is preferably 95 wt% or more, more preferably 98 wt% or more, still more preferably 99 wt% or more.

The chemical purity of the type I crystal of the compound (1) produced by the dissolution-crystallization method is 90% or more. The purity is preferably 95% or more, more preferably 97% or more, still more preferably 98% or more, still further more preferably 99%.

### Type I Crystal of (S)-N-(4-Amino-6-methyl-5-(quinolin-3-yl)-8,9-dihydropyrimido[5,4-b]indolizin-8-yl)acrylamide (Compound (1))

According to one aspect of the present invention, there is provided a type I crystal of the compound (1). The type I crystal of the compound (1) in one embodiment of the present invention obtained by the above-mentioned slurry method has a powder X-ray diffraction spectrum shown in FIG. 1 or a powder X-ray diffraction spectrum substantially the same as the spectrum.

Herein, a characteristic peak in the powder X-ray diffraction spectrum of the type I crystal of the compound (1) in one embodiment of the present invention may be, for example, one or more peaks at diffraction angles (2θ±0.2°) selected from 8.0°, 10.6°, 12.2°, 15.1°, 16.6°, 17.6°, 19.4°, 21.7°, and 26.1°.

The type I crystal of the compound (1) in one embodiment of the present invention may have, in its powder X-ray diffraction spectrum, peaks at 2 or more diffraction angles (2θ±0.2°) selected from, for example, 8.0°, 10.6°, 12.2°, 15.1°, 16.6°, 17.6°, 19.4°, 21.7°, and 26.1°.

The type I crystal of the compound (1) in one embodiment of the present invention may have, in its powder X-ray diffraction spectrum, peaks at 3 or more diffraction angles (2θ±0.2°) selected from, for example, 8.0°, 10.6°, 12.2°, 15.1°, 16.6°, 17.6°, 19.4°, 21.7°, and 26.1°.

The type I crystal of the compound (1) in a preferred embodiment of the present invention has, in its powder X-ray diffraction spectrum, peaks at 4 or more, preferably 5 or more, more preferably 6 or more, still more preferably 7 or more diffraction angles (2θ±0.2°) selected from 8.0°, 10.6°, 12.2°, 15.1°, 16.6°, 17.6°, 19.4°, 21.7°, and 26.1°. In another embodiment of the present invention, the type I crystal of the compound (1) may have, in the powder X-ray diffraction spectrum, peaks at 8 diffraction angles (2θ±0.2°) selected from the foregoing or at all the angles. In still another embodiment, the crystal may further have peaks at one or more diffraction angles (2θ±0.2°) described in Examples except the foregoing.

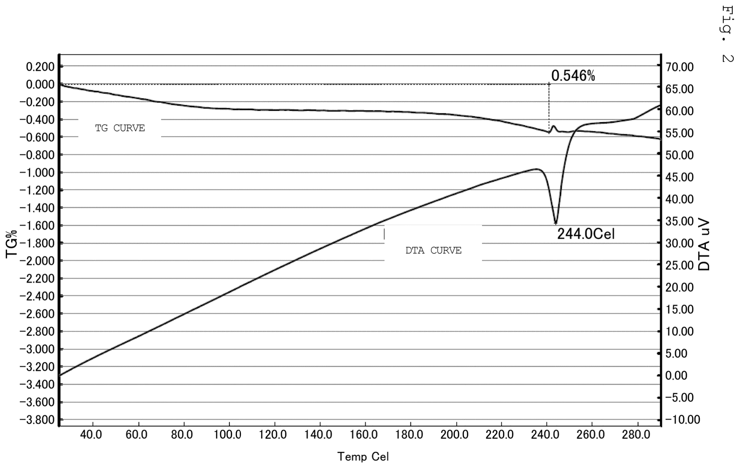

The type I crystal of the compound (1) in one embodiment of the present invention has an endothermic peak (peak top value) determined by thermogravimetry-differential thermal analysis at from 239°C to 249°C, in other words, around 244°C. In another embodiment, the type I crystal of the compound (1) has a thermogravimetry-differential thermal analysis (TG-DTA) curve shown in FIG. 2.

The type I crystal of the compound (1) in one embodiment of the present invention has, in its powder X-ray diffraction spectrum, peaks at 1 or more diffraction angles (2θ±0.2°) selected from 8.0°, 10.6°, 12.2°, 15.1°, 16.6°, 17.6°, 19.4°, 21.7°, and 26.1°, and has the endothermic peak (peak top value) determined by the thermogravimetry-differential thermal analysis at around 244°C. The type I crystal of the compound (1) in a preferred embodiment of the present invention is a crystal having, in the powder X-ray diffraction spectrum, peaks at 2 or more diffraction angles (2θ±0.2°) selected from the foregoing, and having the endothermic peak (peak top value) determined by the thermogravimetry-differential thermal analysis at around 244°C. The type I crystal of the compound (1) in a preferred embodiment of the present invention is a crystal having, in the powder X-ray diffraction spectrum, peaks at 3 or more diffraction angles (2θ±0.2°) selected from the foregoing, and having the endothermic peak (peak top value) determined by the thermogravimetry-differential thermal analysis at around 244°C. The type I crystal of the compound (1) in a preferred embodiment of the present invention is a crystal having, in the powder X-ray diffraction spectrum, peaks at 4 or more diffraction angles (2θ±0.2°) selected from the foregoing, and having the endothermic peak (peak top value) determined by the thermogravimetry-differential thermal analysis at around 244°C, preferably a crystal having peaks at 5 or more diffraction angles (2θ±0.2°) selected from the foregoing and having the endothermic peak (peak top value) determined by the thermogravimetry-differential thermal analysis at around 244°C, more preferably a crystal having peaks at 6 or more diffraction angles (2θ±0.2°) selected from the foregoing and having the endothermic peak (peak top value) determined by the thermogravimetry-differential thermal analysis at around 244°C, still more preferably a crystal having peaks at 7 or more diffraction angles (2θ±0.2°) selected from the foregoing and having the endothermic peak (peak top value) determined by the thermogravimetry-differential thermal analysis at around 244°C. In another embodiment of the present invention, the type I crystal of the compound (1) may have, in the powder X-ray diffraction spectrum, peaks at 8 diffraction angles (2θ±0.2°) selected from the foregoing or at all the angles, and have the endothermic peak (peak top value) determined by the thermogravimetry-differential thermal analysis at around 244°C.

### (Activity and Application)

The crystal of the compound (1) in one embodiment of the present invention has excellent EGFR inhibition activity, and is hence useful as an antitumor agent. In addition, the crystal has an advantage in that the crystal has excellent selectivity for EGFR, and is hence reduced in side effect by any other kinase. Although the kind of a malignant tumor to be treated with the crystal is not particularly limited, examples thereof include an epithelial cancer (e.g., a respiratory cancer, a digestive cancer, a genital cancer, or a secretory cancer), a sarcoma, a hematopoietic tumor, a central nervous tumor, and a peripheral nervous tumor. Of those, an epithelial cancer is preferred, and a respiratory cancer is more preferred. In addition, although the kind of an organ in which a tumor occurs is also not particularly limited, examples thereof include head and neck cancer, esophageal cancer, gastric cancer, colon cancer, rectal cancer, liver cancer, gallbladder and bile duct cancer, biliary tract cancer, pancreatic cancer, lung cancer, breast cancer, ovarian cancer, cervical cancer, uterine body cancer, kidney cancer, bladder cancer, prostate cancer, a testicular tumor, a bone and soft tissue sarcoma, blood cancer, multiple myeloma, skin cancer, a brain tumor, and mesothelioma. Of those, head and neck cancer, gastric cancer, colon cancer, rectal cancer, liver cancer, pancreatic cancer, lung cancer, breast cancer, ovarian cancer, kidney cancer, or prostate cancer is preferred, head and neck cancer, lung cancer, or a brain tumor is particularly preferred, and lung cancer is more preferred.

Further, the crystal of the compound of the present invention or a salt thereof has excellent inhibition activity on a mutant EGFR. Examples of such mutant EGFR include a drug-resistant mutant EGFR and a high-sensitivity mutant EGFR. Accordingly, the crystal of the compound of the present invention or the salt thereof is useful as an antitumor agent even for the above-mentioned malignant tumor having a mutant EGFR.

The crystal of the present invention may be used in postoperative adjuvant chemotherapy to be performed for preventing the recurrence of a tumor after its surgical extraction, or in preoperative adjuvant chemotherapy to be performed in advance for surgically extracting a tumor.

The term "effective dose" of the compound as used herein refers to the amount (therapeutically effective dose) of the compound of the present invention required for such a biological or medical response in a subject as described below: a reduction in, or the inhibition of, enzyme or protein activity is caused; or a symptom is improved, a condition is alleviated, and the progression of a disease is made slower or delayed.

The term "subject" as used herein encompasses a mammalian animal and a nonmammalian animal. In one embodiment, the subject is a human, and may be a human diagnosed as follows: treatment for a symptom, a condition, or a disease disclosed herein is required.

When the crystal or cocrystal of the compound (1) or the salt thereof is used as a drug, various administration forms may be adopted in accordance with treatment purposes with or without the pulverization of the crystal, and the crystal may be used in a dosage form generally utilized as a drug. The form may be any one of, for example, an oral agent, such as a tablet, a capsule, a granule, a fine granule, a powder, or a dry syrup, or a parenteral agent, such as a suppository, an inhalant, a nasal drop, an ointment, a patch, or an injection. A pharmaceutical composition suitable for those administration forms may be produced through use of a pharmaceutically acceptable carrier by a formulation method known to and commonly used by a person skilled in the art.

One embodiment of the present invention provides an antitumor agent for oral administration including the type I crystal of the compound (1) described above. In addition, one embodiment of the present invention provides a method of treating a tumor, the method including orally administering an effective dose of the type I crystal of the compound (1) described above to a subject in need thereof. In addition, according to one embodiment of the present invention, there is provided a use of the type I crystal of the compound (1) described above for the production of an antitumor agent for oral administration. In addition, according to one embodiment of the present invention, there is provided the type I crystal of the compound (1) described above for use in the treatment of a tumor by oral administration.

According to one embodiment of the present invention, there is provided a pharmaceutical composition including the type I crystal of the compound (1) described above. The pharmaceutical composition of one embodiment of the present invention includes the type I crystal of the compound (1) described above and a pharmaceutically acceptable carrier. In addition, according to one embodiment of the present invention, there is provided a use of the type I crystal of the compound (1) described above for the production of a pharmaceutical composition. According to another embodiment of the present invention, there is provided the type I crystal of the compound (1) described above for use as a drug.

Various organic or inorganic carrier substances commonly used as formulation materials are each used as the pharmaceutically acceptable carrier. A formulation material to be used in a solid formulation is, for example, an excipient, a binder, a disintegrator, a lubricant, or a coating agent, and a formulation material to be used in a liquid formulation is, for example, a solvent, a solubilizer, a suspending agent, a tonicity-adjusting agent, a buffer, or a soothing agent. In addition, those formulation materials are each blended at the time of the production of a formulation. In addition, a formulation additive, such as an antiseptic, an antioxidant, a coloring agent, a sweetener, or a stabilizing agent, may be used as required.

Examples of the excipient include a starch, a sugar, a polysaccharide, and an inorganic compound. Examples of the starch include potato starch, corn starch, rice starch, and a partly pregelatinized starch. Examples of the sugar include a monosaccharide, a disaccharide, a trisaccharide, and a sugar alcohol. Examples thereof include lactose, saccharose, trehalose, D-mannitol, raffinose, xylitol, and erythritol. Examples of the sugar also include polysaccharides. Examples thereof include cellulose and dextran, and more specific examples thereof include crystalline cellulose, hydroxypropyl cellulose, and hydroxypropyl methylcellulose. Examples of the inorganic compound include silicic acids, and examples thereof include light anhydrous silicic acid and calcium silicate.

Examples of the binder include hydroxypropyl cellulose, methyl cellulose, polyvinylpyrrolidone, polyvinyl alcohol, maltose syrup powder, and hypromellose.

Examples of the disintegrator include sodium starch glycolate, carmellose calcium, sodium croscarmellose, crospovidone, low-substituted hydroxypropyl cellulose, and a partly pregelatinized starch.

Examples of the lubricant include talc, magnesium stearate, a sucrose fatty acid ester, stearic acid, and sodium stearyl fumarate.

Examples of the coating agent include ethyl cellulose, an aminoalkyl methacrylate copolymer RS, hypromellose, and saccharose.

Examples of the solvent include water, propylene glycol, and physiological saline.

Examples of the solubilizer include alcohols, such as polyethylene glycol and ethanol, cyclodextrin, a cyclodextrin derivative, an ionic surfactant, and a nonionic surfactant, and examples thereof include a sorbitan fatty acid ester such as Polysorbate 80, a sucrose fatty acid ester, and sodium lauryl sulfate.

Examples of the suspending agent include carrageenan, crystalline cellulose-carmellose sodium, polyoxyethylene hydrogenated castor oil, gum arabic, and sodium alginate.

Examples of the tonicity-adjusting agent include sodium chloride, glycerin, and potassium chloride.

Examples of the pH adjuster or the buffer include sodium citrate, hydrochloric acid, lactic acid, phosphoric acid, and sodium dihydrogen phosphate.

Examples of the soothing agent include procaine hydrochloride and lidocaine.

Examples of the antiseptic include ethyl p-oxybenzoate, cresol, and benzalkonium chloride.

Examples of the antioxidant include sodium sulfite, ascorbic acid, and tocopherol.

Examples of the coloring agent include titanium oxide, iron sesquioxide, Food Blue No. 1, and copper chlorophyll.

Examples of a flavoring agent include aspartame, saccharin, sucralose, l-menthol, and mint flavor.

Examples of the stabilizing agent include sodium pyrosulfite, sodium edetate, erythorbic acid, magnesium oxide, and dibutylhydroxytoluene.

When an oral agent is prepared, a tablet, a coated tablet, a granule, a powder, a capsule, or the like may be produced by an ordinary method after the addition of an excipient, and as required, a binder, a disintegrator, a lubricant, a coloring agent, a flavoring agent, or the like to the type I crystal of the compound (1).

When an injection is prepared, injections for subcutaneous, intramuscular, and intravenous uses may each be produced by an ordinary method through the addition of a pH adjuster or buffer, a stabilizing agent, a tonicity-adjusting agent, a local anesthetic, or the like to the type I crystal of the compound (1).

The amount of the type I crystal of the compound (1) to be blended into each administration unit form varies depending on, for example, the symptom of a patient to whom the crystal is to be applied, or the dosage form thereof, and is hence not constant. However, in general, the amount per administration unit form is desirably set to from about 1 mg to about 400 mg in an oral agent, to from about 5 mg to about 300 mg in an injection, or to from about 1 mg to about 400 mg in a suppository or an external preparation in terms of the free base of the compound (1).

In addition, the dose of the type I crystal of the compound (1) of a drug having each administration form per day varies depending on the symptom, body weight, age, sex, or the like of a patient, and hence cannot be unconditionally determined. However, the dose in a normal adult (having a body weight of 50 kg) per day only needs to be set to from about 1 mg to about 1,000 mg, preferably from 1 mg to 400 mg in terms of the free base of the compound (1).

### Examples

The present invention is more specifically described below by way of Examples, but the present invention is by no means limited by these examples. Although the present invention is sufficiently described by Examples, it is understood that various changes and/or modifications can probably be made by a person skilled in the art. Accordingly, such changes and/or modifications are included in the present invention unless such changes and/or modifications deviate from the scope of the present invention.

In the following examples of compounds, the term "%" means "wt%" unless otherwise stated.

### Powder X-ray Diffractometry

Powder X-ray diffractometry was performed in accordance with any one of the following test conditions after an appropriate amount of a test substance had been lightly pulverized in an agate mortar as required.
Apparatus: EMPYREAN (method A) manufactured by PANalytical Reflection method (focusing method)
Target: Cu
X-ray tube current: 40 mA
X-ray tube voltage: 45 kV
Scan range: 2θ=5.0° to 40.0°
Step: 2θ=0.0131°
Average time/step: 8.670 s
Scan speed: 0.0015°/s
Divergence slit: 1°
Scattering slit: 2.0 mm
Light-receiving slit: 8.0 mm
Apparatus: EMPYREAN (method B) manufactured by PANalytical Transmission method
Target: Cu
X-ray tube current: 40 mA
X-ray tube voltage: 45 kV
Scan range: 2θ=2.0° to 40.0°
Step: 2θ=0.0066°
Average time/step: 8.670 s
Scan speed: 0.0008°/s
Divergence slit: 1/2°
Scattering slit: 2.0 mm
Light-receiving slit: Absent

The handling of each apparatus including data processing followed a method and a procedure instructed in the apparatus. Numerical values obtained from various spectra may fluctuate to some extent owing to, for example, the direction of crystal growth, a particle size, or measurement conditions. Accordingly, those numerical values should not be strictly construed.

Thermogravimetry-differential thermal analysis (TG-DTA measurement) was performed for from 4 mg to 5 mg of a test substance in accordance with the following test conditions.
Apparatus: TG/DTA7200
manufactured by Hitachi High-Tech Science Corporation Sample container: made of aluminum
Rate of temperature increase: a temperature is increased from 25°C to 290°C at 10°C/min.
Ambient gas: air (200 mL/min)
Control substance: an empty pan

The handling of each apparatus including data processing followed a method and a procedure instructed in the apparatus.

### Example 1 Production of Type I Crystal of (S)-N-(4-Amino-6-methyl-5-(quinolin-3-yl)-8,9-dihydropyrimido[5,4-b]indolizin-8-yl)acrylamide (Compound (1))

### (Slurry Method)

Acetonitrile (32 mL) was added to (S)-N-(4-amino-6-methyl-5-(quinolin-3-yl)-8,9-dihydropyrimido[5,4-b]indolizin-8-yl)acrylamide (compound (1)) (6.40 g) synthesized by the method described in Patent Document 1, and the mixture was stirred at 50°C for 24 hours. The mixture was left standing to cool to room temperature, and was then stirred under room temperature for 48 hours. After the completion of the stirring, the resulting solid was collected by filtration, and was washed with acetonitrile (6.4 mL), followed by drying under reduced pressure at 50°C for 24 hours. Thus, the crystal (4.14 g) was obtained.

The powder X-ray diffraction spectrum (method A) of the type I crystal obtained by the above-mentioned method was obtained by the above-mentioned procedure, and was shown in FIG. 1. Characteristic diffraction angles observed in the powder X-ray diffraction spectrum include the following peaks.

Characteristic diffraction angles (2θ±0.2°): 8.0°, 10.6°, 12.2°, 15.1°, 16.6°, 17.6°, 19.4°, 21.7°, and 26.1°

Other peaks were as shown in Table 1 below.

**[Table 1]**

| Peak position [°2θ] | Intensity [cts] |
|---|---|
| 5.4 | 2 |
| 8.0 | 420 |
| 10.6 | 306 |
| 12.2 | 872 |
| 13.6 | 45 |
| 14.2 | 189 |
| 15.1 | 383 |
| 15.9 | 444 |
| 16.6 | 642 |
| 17.6 | 743 |
| 19.4 | 2,038 |
| 20.3 | 347 |
| 20.8 | 280 |
| 21.7 | 430 |
| 22.5 | 297 |
| 23.7 | 551 |
| 24.2 | 912 |
| 25.5 | 480 |
| 26.1 | 1,308 |
| 26.9 | 700 |
| 28.8 | 116 |
| 29.3 | 217 |
| 30.2 | 239 |
| 30.7 | 106 |
| 31.7 | 162 |
| 32.0 | 123 |
| 33.4 | 132 |
| 34.1 | 226 |
| 34.5 | 144 |
| 36.0 | 69 |
| 36.7 | 74 |
| 37.6 | 61 |
| 38.7 | 116 |

The thermogravimetry-differential thermal analysis curve of the crystal obtained in Example 1 was obtained by the above-mentioned procedure, and was shown in FIG. 2. An endothermic peak (peak top value) in the thermogravimetry-differential thermal analysis curve was observed at around 244°C.

### Preliminary Test Investigation of Conditions for Production of Crystal of (S)-N-(4-Amino-6-methyl-5-(quinolin-3-yl)-8,9-dihydropyrimido[5,4-b]indolizin-8-yl)acrylamide (Compound (1))

A preliminary test was performed for the purpose of searching for a solvent appropriate for the production of a crystal of (S)-N-(4-amino-6-methyl-5-(quinolin-3-yl)-8,9-dihydropyrimido[5,4-b]indolizin-8-yl)acrylamide (compound (1)). The precipitation of the crystal and conditions therefor were investigated by changing the kinds and mixing ratio of solvents. A yield at that time was determined by a calculated value. The results were shown in Table 2 below. The precipitation of the crystal was visually observed.

The solvents were marked down by the preliminary test, and Examples 2 and 3 to be described later were performed.

**[Table 2]**

| Solvent | Aging temperature (°C) | Aging time (h) | Yield (%) |
|---|---|---|---|
| Ethanol/water=2/1 | 25 | 19 | - |
| Ethanol/water=1.5/1 | | 19 | 35 |
| Ethanol/water=1/1 | | 3 | 15 |
| Ethanol/water=1/1 | | 16 | 52 |
| Ethanol/water=0.67/1 | | 16 | 80 |
| Ethanol/water=0.5/1 | | 2 | 94 |
| 1-Propanol/water=1/1 | 25 | 16 | - |
| 1-Propanol/water=0.67/1 | | 16 | 19 |
| 1-Propanol/water=0.5/1 | | 16 | 41 |
| 1-Propanol/water=0.33/1 | | 16 | 87 |
| 2-Propanol/water=2/1 | 25 | 16 | - |
| 2-Propanol/water=1.5/1 | | 16 | - |
| 2-Propanol/water=1/1 | | 40 | 21 |
| 2-Propanol/water=0.67/1 | | 16 | 62 |
| 2-Propanol/water=0.5/1 | | 16 | 87 |
| Methanol/water=2/1 | 25 | 16 | 65 |
| Methanol/water=1.5/1 | | 16 | 81 |
| Methanol/water=1/1 | | 16 | 88 |
| Acetonitrile/water=1.5/1 | 25 | 16 | - |
| Acetonitrile/water=1/1 | | 16 | - |
| Acetonitrile/water=0.67/1 | | 16 | 59 |
| Acetonitrile/water=0.5/1 | | 16 | 77 |
| Acetonitrile/water=0.33/1 | | 16 | 90 |

| | | | |
|---|---|---|---|
| -: No crystal precipitation could be observed. | | | |

### Example 2 Production of Type I Crystal of (S)-N-(4-Amino-6-methyl-5-(quinolin-3-yl)-8,9-dihydropyrimido[5,4-b]indolizin-8-yl)acrylamide (Compound (1))

### (Dissolution-crystallization Method)

75% Ethanol water (15 mL) was added to (S)-N-(4-amino-6-methyl-5-(quinolin-3-yl)-8,9-dihydropyrimido[5,4-b]indolizin-8-yl)acrylamide (compound (1)) (1.00 g) synthesized by the method described in Patent Document 1, and the mixture was stirred at 55°C for 1 hour. After that, insoluble matter was separated by filtration, and was washed with 75% ethanol water (0.6 mL). Next, water (16.6 mL) was added to the filtrate, and the mixture was stirred at 55°C for 2 hours. After that, the mixture was cooled to 25°C, and was stirred for 20 hours. The resulting solid was collected by filtration, and was washed with 36% ethanol water (10 mL), followed by drying under reduced pressure at 50°C for 5 hours. Thus, the crystal (825 mg, yield: 83%, chemical purity: 99.6%) was obtained.

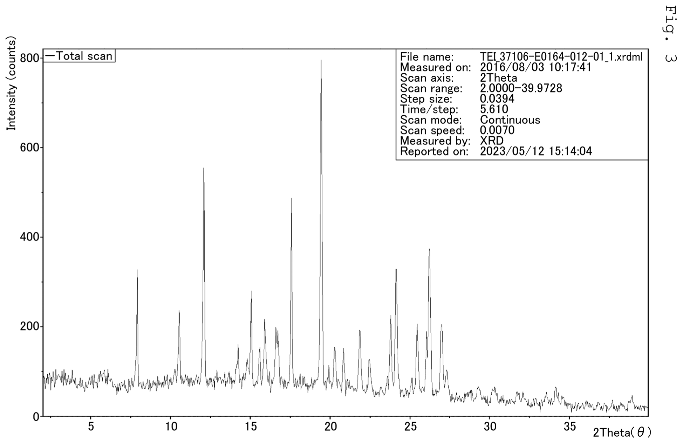

The powder X-ray diffraction spectrum (method A) of the type I crystal obtained by the above-mentioned method was obtained by the above-mentioned procedure, and was shown in FIG. 3. Characteristic diffraction angles observed in the powder X-ray diffraction spectrum include the following peaks.

Characteristic diffraction angles (2θ±0.2°): 8.0°, 10.6°, 12.2°, 15.1°, 16.6°, 17.6°, 19.4°, 21.7°, and 26.1°

### Example 3 Production of Type I Crystal of (S)-N-(4-Amino-6-methyl-5-(quinolin-3-yl)-8,9-dihydropyrimido[5,4-b]indolizin-8-yl)acrylamide (Compound (1))

### (Dissolution-crystallization Method)

Production was performed by the same operations as those of the method described in Example 2 except that the solvent ratio of a mixed solvent was changed as shown in the table (ratio shown in Table 3).

A type I crystal of (S)-N-(4-amino-6-methyl-5-(quinolin-3-yl)-8,9-dihydropyrimido[5,4-b]indolizin-8-yl)acrylamide (compound (1)) having a high chemical purity (99% or more) was able to be produced even when the ratio of the mixed solvent was changed.

**[Table 3]**

| Solvent | | Coolin g rate (°C/h) | Aging temperatur e (°C) | Agin g time (h) | Precipitatio n | Yiel d (%) | Chemica l purity (%) |
|---|---|---|---|---|---|---|---|
| Ethanol/water=0.67/1 | 40 % | 13 | 25 | 17 | ○ | 73 | 99.73 |
| Ethanol/water=0.57/1 | 36 % | 13 | 25 | 20 | ○ | 82 | 99.66 |
| Ethanol/water=0.57/1 | 36 % | 8 | 25 | 17 | ○ | 78 | 99.70 |
| Ethanol/water=0.57/1 | 36 % | 13*** | 25 | 16 | ○ | 80 | 99.65 |
| Ethanol/water=0.5/1 | 33 % | 40 | 25 | 17 | ○ | 88 | 99.58 |
| Ethanol/water=0.5/1 | 33 % | 13 | 25 | 19 | ○ | 85 | 99.62 |
| Ethanol/water=0.5/1 | 33 % | 8 | 25 | 12 | ○ | 83 | 99.65 |
| Ethanol/water=0.5/1 | 33 % | 8 | 25 | 15 | ○ | 85 | 99.62 |
| Ethanol/water=0.5/1 | 33 % | 13 | 25 | 19 | ○ | 88 | 99.56 |
| Ethanol/water=0.5/1 | 33 % | 13 | 25 | 19 | ○ | 86 | 99.59 |
| Ethanol/water=0.5/1 | 33 % | 13 | 25 | 12 | ○ | 83 | 99.65 |
| Ethanol/water=0.5/1 | 33 % | 13*** | 25 | 15 | ○ | 84 | 99.59 |
| Ethanol/water=0.5/1 | 33 % | 13 | 25 | 12 | ○ | 82 | 99.70 |
| Ethanol/water=0.5/1 | 33 % | 13 | 25 | 17 | ○ | 79 | 99.69 |
| Acetonitrile/water=0.5 /1 | 33 % | 40 | 25 | 18 | ○ | 62 | 99.68 |
| Acetonitrile/water=0.5 /1 | 33 % | 8 | 25 | 17 | ○ | 68 | 99.67 |

| | | | | | | | |
|---|---|---|---|---|---|---|---|
| *** The mixture was kept at 55°C for 1 hour, and was then cooled to 25°C at a rate of 13°C/h. | | | | | | | |

### Comparative Example 1 Production of Solvate Crystal Type "b" of (S)-N-(4-Amino-6-methyl-5-(quinolin-3-yl)-8,9-dihydropyrimido[5,4-b]indolizin-8-yl)acrylamide (Compound (1))

A type I crystal (1.5 g) of the compound (1) was dissolved in methanol (225 mL), and then the solvent was dried with an evaporator and a hot water bath at 40°C under reduced pressure. Thus, 1.012 g of an amorphous solid of the compound (1) was obtained.

Anisole (20 mL) was added to 1 g of the amorphous solid, and the mixture was stirred at room temperature for about 28 hours. After that, the mixture was filtered under reduced pressure, and the solid was recovered. The solid was dried under reduced pressure at room temperature for about 26 hours to provide 856 mg of the title crystal.

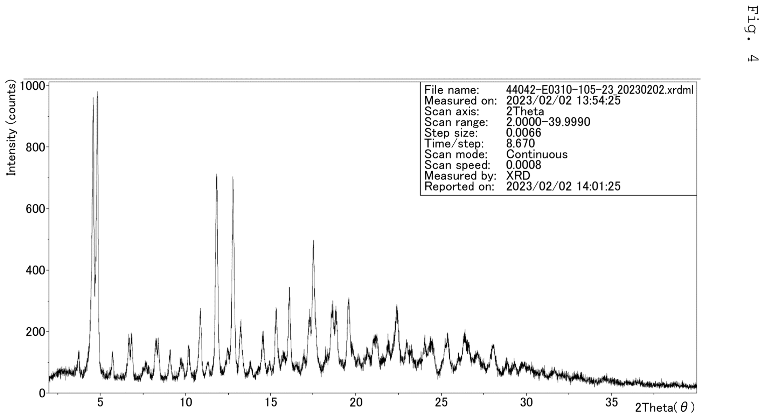

The powder X-ray diffraction spectrum (method B) of the resultant crystal was obtained by the above-mentioned procedure, and was shown in FIG. 4. Characteristic diffraction angles observed in the powder X-ray diffraction spectrum were as described below.

Characteristic diffraction angles (2θ±0.2°): 10.9°, 11.8°, 12.8°, 15.3°, 16.1°, 19.6°, and 22.4°

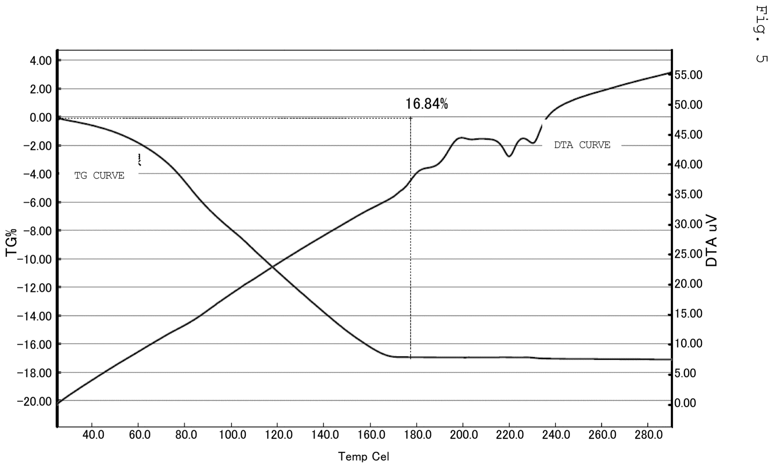

The thermogravimetry-differential thermal analysis curve of the resultant crystal was obtained by the above-mentioned procedure, and was shown in FIG. 5.

### Reference Example 1 Production of Amorphous Solid of (S)-N-(4-Amino-6-methyl-5-(quinolin-3-yl)-8,9-dihydropyrimido[5,4-b]indolizin-8-yl)acrylamide (Compound (1))

The type I crystal (500 mg) of the compound (1) was dissolved in the mixed solvent of ethyl acetate (80 mL) and methanol (40 mL) at 40°C, and then the mixture was dried with an evaporator and a hot water bath at 40°C under reduced pressure. Thus, 279.6 mg of an amorphous solid of the compound (1) was obtained.

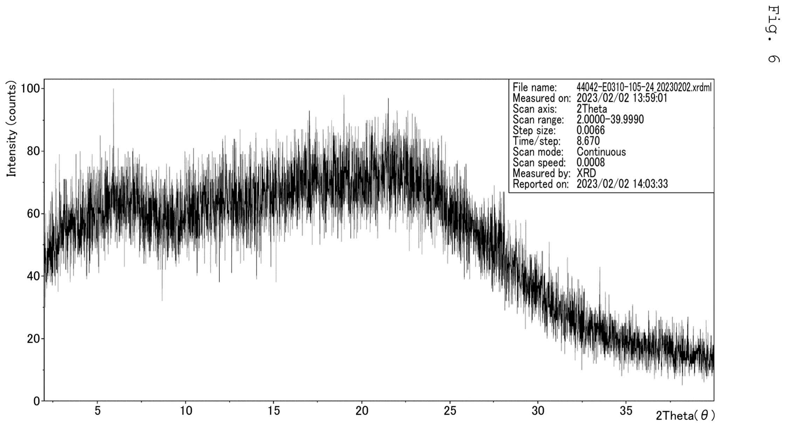

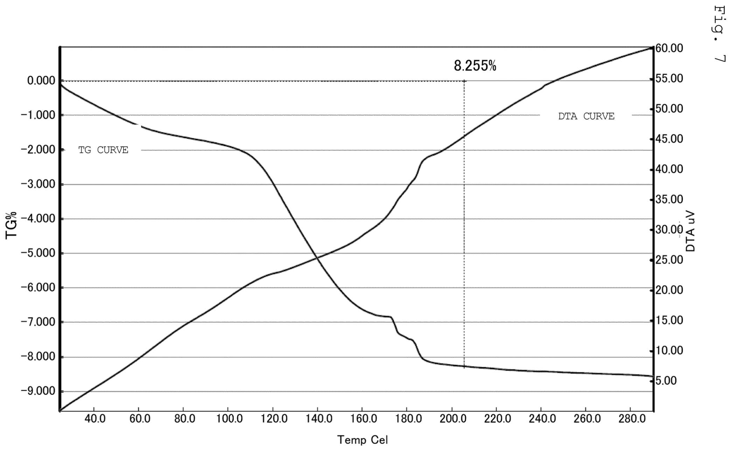

The powder X-ray diffraction spectrum (method B) of the resultant amorphous solid was obtained by the above-mentioned procedure, and was shown in FIG. 6. As a result of the measurement, no characteristic diffraction angles were observed. The thermogravimetry-differential thermal analysis curve of the resultant amorphous solid was obtained by the above-mentioned procedure, and was shown in FIG. 7.

### Test Example 1 Solid Stability Test

A temperature influence was tested by using the type I crystal, solvate crystal type "b", and amorphous solid of the compound (1) obtained in Example 1, Comparative Example 1, and Reference Example 1, respectively. An evaluation was performed by the following procedure. In addition, with regard to the appearances of the crystals and the amorphous solid, the states thereof before and after their storage were visually observed.
Storage condition: 60°C or 80°C (closed system) (HIFLEX (high-temperature storage machine manufactured by ETAC Engineering Co., Ltd.))
Storage period: 2 weeks and 4 weeks
Storage amount: about 100 mg
Storage container: a glass bottle

The results are shown in Table 4 and Table 5 below.

**[Table 4]**

| | At the beginning | 2 Weeks after | | | | 4 Weeks after | | | |
|---|---|---|---|---|---|---|---|---|---|
| Storage at 60°C | Purity | Purity | Change amount | Crystal form | Color tone change | Purity | Change amount | Crystal form | Color tone change |
| Type I crystal | 98.84% | 98.90% | 0.06% | No change | Absent | 98.86% | 0.02% | No change | Absent |
| Solvate crystal type "b" | 98.76% | 88.16% | -10.60% | No change | Absent | 87.21% | -11.55% | No change | Absent |
| Amorphous solid | 98.75% | 98.09% | -0.66% | No change | Absent | 97.90% | -0.85% | No change | Absent |

**[Table 5]**

| | At the beginnin g | 2 Weeks after | | | | 4 Weeks after | | | |
|---|---|---|---|---|---|---|---|---|---|
| Storage at 80°C | Purity | Purit y | Chang e amoun t | Crysta 1 form | Color tone change | Purit y | Chang e amoun t | Crysta 1 form | Color tone change |
| Type I crystal | 98.84% | 98.89 % | 0.05% | No change | Absent | 98.91 % | 0.07% | No change | Absent |
| Solvate crystal type "b" | 98.76% | 84.57 % | - 14.49 % | No change | Absent | 83.38 % | 15.38 | No change | Absent |
| Amorphou s solid | 98.75% | 92.37 % | 6.38% | No change | Colorin g | 90.85 | 7.90% | No change | Colorin g |

A change in amount of related substances (amount of substances detected in addition to the compound (1)) was analyzed by HPLC in accordance with the following method. A product obtained as follows was used as a sample for analysis: about 1 mg of each sample was weighed as the compound (1); the sample was dissolved in 10 mL of a mixed liquid of acetonitrile and water (3:1 v/v); and 5 µL of the liquid was accurately weighed out.

### HPLC Measurement Method (Stability Test)

The amount of the related substances in the sample solution was measured by HPLC analysis. The handling of each apparatus including data processing followed a method and a procedure instructed in the apparatus.
Column: InertSustain C18HP (4.6 mm×150 mm, 3 µm) manufactured by GL Sciences Inc.
UV detection: 220 nm
Column temperature: 40°C
Flow rate: 1.0 mL/min
Sample cooler: 5°C
Sample concentration: 0.1 mg/mL
Mobile phase A: 0.1% phosphoric acid
Mobile phase B: acetonitrile

Gradients are shown in Table 6.

**[Table 6]**

| Time (min) | Mobile phase A (%) | Mobile phase B (%) |
|---|---|---|
| 0-30 | 90→50 | 10→50 |
| 30-31 | 50→30 | 50→70 |
| 31-35 | 30 | 70 |
| 35-36 | 30→90 | 70→10 |
| 36-45 | 90 | 10 |

As a result, it was revealed that the type I crystal of the compound (1) was an extremely stable crystal because the chemical purity of the crystal substantially remained unchanged, substantially no increase in amount of related substances was observed, and the crystal form and color tone thereof showed no changes as compared to the solvate crystal type "b" and amorphous solid thereof.

### Test Example 2 Dynamic Vapor Sorption (DVS) Test

A vapor sorption test was performed by using each of the type I crystal, solvate crystal type "b", and amorphous solid of the compound (1) obtained in Example 1, Comparative Example 1, and Reference Example 1, respectively. The vapor sorption test was performed in accordance with the following conditions. About 10 mg of a sample was loaded into a dedicated holder made of quartz, and the weights of the sample at respective humidities were continuously measured and recorded under the following conditions. The handling of each apparatus including data processing followed a method and a procedure instructed in the apparatus.
Apparatus: VTI-SA+ (manufactured by TA Instruments)
Drying temperature: 60°C
Rate of temperature increase: 5°C/min
Drying equilibrium: it is recognized that the weight of the sample does not reduce by 0.01 wt% in 5 minutes for a time period in the range of not more than 300 minutes.
Measurement temperature: 25°C
Humidification equilibrium: it is recognized that the weight does not increase by 0.01 wt% in 5 minutes for a time period in the range of not more than 120 minutes.
Relative humidity program: A humidity is increased from 5%RH to 95%RH in increments of 5%RH, and is reduced from 95%RH to 5%RH in increments of 5%RH.

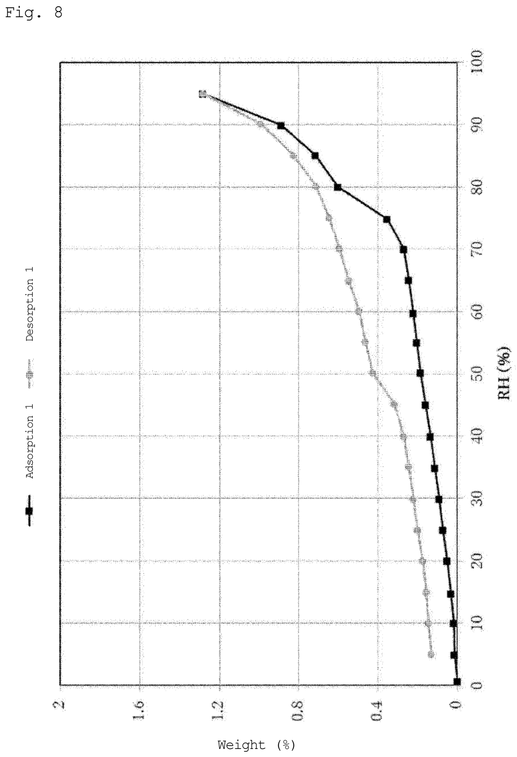

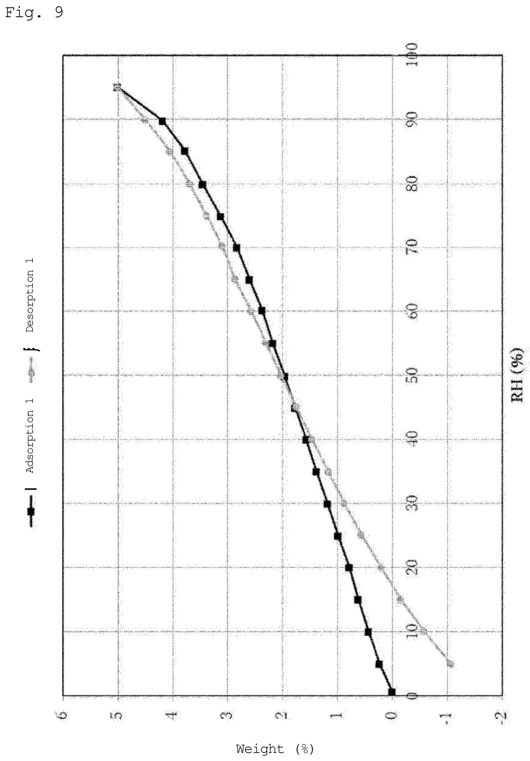

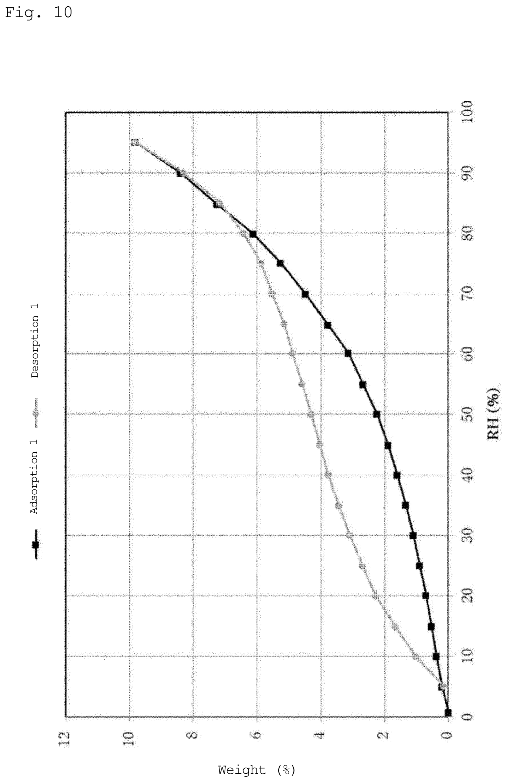

Weight changes in the measurement conditions and ranges obtained by those tests are shown in FIG. 8 to FIG. 10.

As shown in FIG. 8 to FIG. 10, in the vapor desorption test, the type I crystal of the compound (1) showed a weight increase of 1.28% under a relative humidity (RH) of 95%. Further, the type I crystal showed substantially no weight increase (less than 0.4%) even when the humidity was changed up to 75%RH. The solvate crystal type "b" of the compound (1) was recognized to show a weight increase of 5% or more under the same conditions, and a substantially linear weight increase starting from a low-humidity region was observed. The amorphous solid thereof was recognized to show a weight increase of 2% or more at 50%RH, and was recognized to show a weight increase of about 10% at 95%RH.

It can be said from the above-mentioned results that the type I crystal of the compound (1) is less hygroscopic compared to the solvate crystal type "b" and amorphous solid thereof, and is hence excellent among candidate compounds for the development of a drug from the viewpoint of the industrial production of a drug having stable quality.

In addition to Comparative Example 1, a metastable crystal and a hydrate crystal of the compound (1) were obtained and compared with the type I crystal obtained according to the method described in Example 1. The results indicated that the type I crystal obtained according to the method described in Example 1 had better properties in terms of hygroscopicity, storage stability, etc., compared to those of the metastable crystal and hydrate crystal (data not shown) .

Some embodiments of the present invention have been described above. However, those embodiments are presented as examples, and are not intended to limit the scope of the invention. Those novel embodiments may be implemented in other various modes, and various kinds of omissions, replacements, and modifications may be made thereto without departing from the gist of the invention. Those embodiments and modifications thereof are included in the scope and gist of the invention, and are included in the scopes of the invention described in the appended claims and their equivalents.

## Claims

1. A type I crystal of (S)-N-(4-amino-6-methyl-5-(quinolin-3-yl)-8,9-dihydropyrimido[5,4-b]indolizin-8-yl)acrylamide, the crystal having, in a powder X-ray diffraction spectrum thereof measured with a CuKα characteristic X-ray, peaks at 3 or more diffraction angles (2θ±0.2°) selected from the group consisting of: 8.0°; 10.6°; 12.2°; 15.1°; 16.6°; 17.6°; 19.4°; 21.7°; and 26.1°.

2. The crystal according to claim 1, wherein the crystal has, in the powder X-ray diffraction spectrum measured with the CuKα characteristic X-ray, peaks at 5 or more diffraction angles (2θ±0.2°) selected from the group consisting of: 8.0°; 10.6°; 12.2°; 15.1°; 16.6°; 17.6°; 19.4°; 21.7°; and 26.1°.

3. The crystal according to claim 1 or 2, wherein the crystal has, in the powder X-ray diffraction spectrum measured with the CuKα characteristic X-ray, peaks at 7 or more diffraction angles (2θ±0.2°) selected from the group consisting of: 8.0°; 10.6°; 12.2°; 15.1°; 16.6°; 17.6°; 19.4°; 21.7°; and 26.1°.

4. A type I crystal of (S)-N-(4-amino-6-methyl-5-(quinolin-3-yl)-8,9-dihydropyrimido[5,4-b]indolizin-8-yl)acrylamide, the crystal having a powder X-ray diffraction spectrum substantially the same as a powder X-ray diffraction spectrum measured with a CuKα characteristic X-ray, the spectrum being shown in FIG. 1.

5. The crystal according to any one of claims 1 to 4, wherein the crystal has an endothermic peak determined by thermogravimetry-differential thermal analysis at around 244°C.

6. The crystal according to any one of claims 1 to 5, wherein the crystal has a crystal purity of 95 wt% or more.

7. The crystal according to any one of claims 1 to 6, wherein the crystal has a chemical purity of 95% or more.

8. A method of producing the type I crystal of (S)-N-(4-amino-6-methyl-5-(quinolin-3-yl)-8,9-dihydropyrimido[5,4-b]indolizin-8-yl)acrylamide of any one of claims 1 to 7, the method comprising a step of stirring (S)-N-(4-amino-6-methyl-5-(quinolin-3-yl)-8,9-dihydropyrimido[5,4-b]indolizin-8-yl)acrylamide in a solvent containing at least one kind selected from the group consisting of: a lower alcohol; an aprotic polar solvent; and water.

9. A method of producing the type I crystal of (S)-N-(4-amino-6-methyl-5-(quinolin-3-yl)-8,9-dihydropyrimido[5,4-b]indolizin-8-yl)acrylamide of any one of claims 1 to 7, the method comprising a step of dissolving and crystallizing (S)-N-(4-amino-6-methyl-5-(quinolin-3-yl)-8,9-dihydropyrimido[5,4-b]indolizin-8-yl)acrylamide in a mixed solvent of a lower alcohol and water, or a mixed solvent of an aprotic polar solvent and water.

10. A pharmaceutical composition comprising the crystal of any one of claims 1 to 7.
